# EUROPEAN PATENT APPLICATION

(11) **EP 4 588 468 A1**
(43) Date of publication of application: **23.07.2025**
(21) Application number: 22958917.1
(22) Date of filing: 16.12.2022
(51) Int. Cl.: A61K 6/884, C07K 7/06

(54) **COMPOSITION FOR DENTIN ADHESION AND COMPOSITION FOR PULP RESTORATION, EACH COMPOSITION CONTAINING CPNE7-DERIVED PEPTIDE**

(30) Priority: 15.09.2022 KR 20220116563
(71) Applicant: Hysensbio Co., Ltd, Gwacheon-si Gyeonggi-do 13814 (KR); Seoul National University Dental Hospital, Seoul 03080 (KR)
(72) Inventor: PARK, Joo Hwang, Incheon 21986 (KR); GUG, Hye Ri, Seoul 02811 (KR); SHON, Won Jun, Seoul 06584 (KR)
(74) Representative: Mewburn Ellis LLP
(86) International application number: PCT/KR2022/020540
(87) International publication number: WO 2024/058315

(57) **Abstract**

A dentin adhesion composition according to the present invention contains a peptide composed of an amino acid sequence of the following General Formula 1: K-Y-R1-R2-R3-R4-R5-R6-R7-R8 (General Formula 1), in General Formula 1, R1 is arginine (R), lysine (K), or glutamine (Q); R2 is arginine (R) or glutamine (Q); R3, R4, and R5 are each arginine (R) or lysine (K); R6 is asparagine (N) or serine (S); and R7 and R8 are lysine (K) or tyrosine (Y). The composition according to the present invention is applied to the treatment of damaged dentin, such as dental caries or fractures, and thus can prevent possible dentin necrosis, dental extraction, or dental loss after treatment.

## Description

### [Technical Field]

The present invention relates to a dentin adhesive and a composition for pulp restoration, and more specifically, to a dentin adhesive and a composition for pulp restoration, each composition including a CPNE7-derived peptide that forms biocompatible tubular dentin and tubular dentin having a continuous structure.

### [Background Art]

Various dental restorative materials have physical properties (such as thermal expansion coefficient, strength, abrasivity, elasticity, etc.) different from those of the teeth themselves, and the process of physically and chemically adapting the materials to the teeth involves processes such as tooth extraction and chemical treatment. The chemical stimulation and microleakage of dental materials for preservation and prosthetic restoration and the stimulation during the treatment process may deteriorate or cause hypersensitivity and pulp lesions in addition to existing pathological dental problems (such as fractures, hypersensitivity, and caries). This causes necrosis of the pulp of living tissue inside the tooth, leading to root canal treatment to remove the pulp and additional tooth extraction and loss. In addition, when the dentin of the tooth is damaged due to various causes and the dentinal tubules are exposed to the outside, tooth sensitivity symptoms occur. These symptoms may also occur when dentin is removed during a dental treatment process.

**In** order to reduce the tooth sensitivity symptoms due to exposed dentinal tubules, dentinal tubule occlusion and physiological mineralization are required. To overcome these limitations, the concept of physiological dentin sealing was introduced, and a novel restorative material with the physiological activity of promoting the recovery of odontoblasts responsible for dentin secretion is needed.

Recently, geriatric dental diseases due to aging of the population, which has emerged as a serious social problem, are also affecting the dental field due to the expansion of the national health insurance for the elderly population. There are several research results indicating that the number of natural teeth in the elderly tends to decrease with age, and the quality of life decreases as the number of natural teeth decreases. Therefore, there is an increasing demand for techniques and materials for preserving as many of the patient's natural teeth as possible.

The teeth include hard tissues such as enamel, dentin, and cementum, and internal soft tissue such as pulp. Nerves and blood vessels are developed in the pulp, and odontoblasts that form dentin are located at the boundary between the pulp and dentin. When the dentin is damaged due to various reasons such as dental caries or fracture, the symptoms felt by the patient and the necessary treatment technique will vary depending on the degree of damage. When the damage to the tooth is limited to enamel or dentin, the weakened dentin is removed and a restorative material is filled. When the area of the damage is close to the pulp or has invaded the pulp, in the case in which the area of the exposed pulp is small, the pulp is directly sealed and restoration is performed using a pulp restorative material.

In order to restore the structure and function of the teeth from damage, preservative and prosthetic restorative materials composed of various components such as amalgam, glass ionomer cement, composite resins, gold, and porcelain are used. Among these, the material that is most commonly used for direct restoration is resin, and a dentin adhesive is used to attach the resin to the dentin. Currently, 8th-generation adhesives with a shortened application step are the most common, and a representative 8th-generation adhesive is Bisco Dental's All-Bond Universal product. When an adhesive is developed that restores the thickness of damaged dentin during resin restoration, prevents microleakage, and has a biological effect of inducing the restoration of damaged dentin thickness, it will be able to help protect natural teeth in the future, thereby improving overall oral health and quality of life.

When a small area of pulp is exposed or the remaining dentin is thin and close to the pulp, pulp restoration is applied to maintain pulp vitality. The ideal pulp restorative material is one that does not cause inflammation of the pulp, combines with the dentin to prevent microleakage, forms a dentinal bridge, and is convenient for clinical use. Many materials such as calcium hydroxide, mineral trioxide aggregate (MTA), and adhesive resins are being studied for successful pulp restoration, but among them, MTA is being used for the most diverse purposes due to its biocompatibility. One of the important functions of direct pulp restorative materials is the biological effect of stimulating odontoblasts in the pulp to induce new dentin formation. This effect enables sealing of the exposed pulp, preventing the treatment from moving on to endodontic therapy. Since endodontic therapy removes all the pulp inside the tooth, the therapy ultimately makes it difficult to preserve the natural tooth. Therefore, the development of direct pulp restorative materials with the function of inducing dentin formation is also necessary for the promotion of overall oral health.

Resin restorative materials are often used, and resin restorative materials have good adhesion to dentin, but patients often experience discomfort after restoration due to the toxicity of the monomer. In order to minimize the side effects of treatment, it is necessary to seal the exposed dentinal tubules and restore the thickness of the damaged dentin to minimize the effect of the monomer on the pulp. MTA, which is widely used as a pulp restorative material, also has good biocompatibility and sealing power, but it forms a restorative dentin that is different from the existing tubular dentin. In the long term, microleakage of the restorative dentin occurs at the boundary with the existing dentin.

### [Disclosure]

### [Technical Problem]

An object of the present invention is to provide a dentin adhesive that prevents necrosis of the pulp, tooth extraction, or tooth loss that may occur when applying various dental restorative materials to teeth.

Another object of the present invention is to provide a composition for pulp restoration that can be directly applied to seal the pulp and perform restoration when the area of damage to the tooth is close to the pulp or has invaded the pulp, in the case in which the area of the exposed pulp is small.

The objects of the present invention are not limited to those mentioned above, and other objects that are not mentioned will be clearly understood by those skilled in the art to which the present invention pertains, from the description below.

### [Technical Solution]

According to one aspect of the present invention for solving the above-described technical problems, there is provided a composition for dentin adhesion including a peptide consisting of an amino acid sequence of General Formula 1 below:
K-Y-R1-R2-R3-R4-R5-R6-R7-R8 (General Formula 1),
in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

Regarding dentin adhesion, a variant peptide having a sequence differing by one or more amino acid residues from the amino acid sequence constituting the peptide of the present invention is also included in the scope of the peptide provided by the present invention, as long as it can exhibit an effect of promoting the physiological mineralization of dentin.

Generally, amino acid exchanges in proteins and polypeptides that do not change the overall activity of the molecule are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In addition, a peptide whose structural stability against heat, pH, and the like is increased or whose ability to promote dentin synthesis is increased by a mutation or modification in the amino acid sequence may be included.

For example, even when glutamine, an acidic amino acid located at position 3 of the peptide of SEQ ID NO: 1 provided in the present invention, is substituted with lysine or arginine, which are basic amino acids, the effect of the peptide provided in the present invention may still be exhibited; even when arginine, a basic amino acid located at position 4 or 5 of the peptide of SEQ ID NO: 1, is substituted with glutamine, which is an acidic amino acid, or lysine, which is a basic amino acid, the effect of the peptide provided in the present invention may still be exhibited; even when lysine, a basic amino acid located at position 6, 7, or 9 of the peptide of SEQ ID NO: 1, is substituted with arginine, which is a basic amino acid, or tyrosine, which is an aromatic amino acid, the effect of the peptide provided in the present invention may still be exhibited; even when asparagine, an acidic amino acid located at position 8 of the peptide of SEQ ID NO: 1, is substituted with serine, which is a neutral amino acid, the effect of the peptide provided in the present invention may still be exhibited; and even when tyrosine, an aromatic amino acid located at position 10 of the peptide of SEQ ID NO: 1, is substituted with lysine, which is a basic amino acid, the effect of the peptide provided in the present invention may still be exhibited.

In this way, even when the acidic amino acids, basic amino acids, or aromatic amino acids constituting the peptide of the present invention are substituted with different acidic amino acids, basic amino acids, neutral amino acids, or aromatic amino acids, the effect of the peptide provided in the present invention may still be exhibited, so it is obvious that a variant peptide having a sequence that differs by one or more amino acid residues from the amino acid sequence constituting the peptide of the present invention is also included in the scope of the peptide provided in the present invention.

In addition, even when the peptide of the present invention has a form in which any amino acid is added to its N-terminus or C-terminus, the effect of the peptide provided in the present invention may still be exhibited, so this peptide is included in the scope of the peptide provided in the present invention. As an example, the peptide may be in a form in which 1 to 300 amino acids are added to the N-terminus or C-terminus of the peptide, as another example, the peptide may be in a form in which 1 to 100 amino acids are added to the N-terminus or C-terminus of the peptide, and as still another example, the peptide may be in a form in which 1 to 24 amino acids are added to the N-terminus or C-terminus of the peptide.

Specifically, the peptide according to General Formula 1 may be expressed as Tables 1 to 12 below.

For example, a peptide (SEQ ID NO: 1) may be synthesized by the 9-fluorenylmethyloxycarbonyl (Fmoc) method, and amino acids of the synthesized peptide may be substituted to synthesize peptides of each group (Tables 1 to 12).

N-KYQRRKKNKY-C (SEQ ID NO: 1)

First, the peptides of Group 1 may be synthesized by synthesizing the peptide of SEQ ID NO: 1 or by substituting amino acids 5 to 7 of the peptide of SEQ ID NO: 1 with lysine or arginine (Table 1).

**[Table 1]**

| SEQ ID NO. | Amino acid sequence (N-C) |
|---|---|
| 1 | KYQRRKKNKY |
| 2 | KYQRRKRNKY |
| 3 | KYQRRRKNKY |
| 4 | KYQRRRRNKY |
| 5 | KYQRKKKNKY |
| 6 | KYQRKRKNKY |
| 7 | KYQRKKRNKY |
| 8 | KYQRKRRNKY |

Next, the peptides of Group 2 may be synthesized by substituting amino acids 5 to 7 of the peptide of SEQ ID NO: 1 with lysine or arginine and substituting amino acid 8 with serine (Table 2).

**[Table 2]**

| SEQ ID NO. | Amino acid sequence (N-C) |
|---|---|
| 9 | KYQRRKKSKY |
| 10 | KYQRRKRSKY |
| 11 | KYQRRRKSKY |
| 12 | KYQRRRRSKY |
| 13 | KYQRKKKSKY |
| 14 | KYQRKRKSKY |
| 15 | KYQRKKRSKY |
| 16 | KYQRKRRSKY |

Next, the peptides of Group 3 may be synthesized by substituting amino acids 5 to 7 of the peptide of SEQ ID NO: 1 with lysine or arginine and substituting amino acid 9 with tyrosine (Table 3).

**[Table 3]**

| SEQ ID NO. | Amino acid sequence (N-C) |
|---|---|
| 17 | KYQRRKKNYK |
| 18 | KYQRRKRNYK |
| 19 | KYQRRRKNYK |
| 20 | KYQRRRRNYK |
| 21 | KYQRKKKNYK |
| 22 | KYQRKRKNYK |
| 23 | KYQRKKRNYK |
| 24 | KYQRKRRNYK |

Next, the peptides of Group 4 may be synthesized by substituting amino acids 5 to 7 of the peptide of SEQ ID NO: 1 with lysine or arginine, substituting amino acid 8 with serine, substituting amino acid 9 with tyrosine, and substituting amino acid 10 with lysine (Table 4).

**[Table 4]**

| SEQ ID NO. | Amino acid sequence (N-C) |
|---|---|
| 25 | KYQRRKKSYK |
| 26 | KYQRRKRSYK |
| 27 | KYQRRRKSYK |
| 28 | KYQRRRRSYK |
| 29 | KYQRKKKSYK |
| 30 | KYQRKRKSYK |
| 31 | KYQRKKRSYK |
| 32 | KYQRKRRSYK |

Next, the peptides of Group 5 may be synthesized by substituting amino acid 3 of the peptide of SEQ ID NO: 1 with arginine, amino acid 4 with glutamine, and amino acids 5 to 7 with lysine or arginine (Table 5).

**[Table 5]**

| SEQ ID NO. | Amino acid sequence (N-C) |
|---|---|
| 33 | KYRQRKKNKY |
| 34 | KYRQRKRNKY |
| 35 | KYRQRRKNKY |
| 36 | KYRQRRRNKY |
| 37 | KYRQKKKNKY |
| 38 | KYRQKRKNKY |
| 39 | KYRQKKRNKY |
| 40 | KYRQKRRNKY |

Next, the peptides of Group 6 may be synthesized by substituting amino acid 3 of the peptide of SEQ ID NO: 1 with arginine, amino acid 4 with glutamine, amino acids 5 to 7 with lysine or arginine, and amino acid 8 with serine (Table 6).

**[Table 6]**

| SEQ ID NO. | Amino acid sequence (N-C) |
|---|---|
| 41 | KYRQRKKSKY |
| 42 | KYRQRKRSKY |
| 43 | KYRQRRKSKY |
| 44 | KYRQRRRSKY |
| 45 | KYRQKKKSKY |
| 46 | KYRQKRKSKY |
| 47 | KYRQKKRSKY |
| 48 | KYRQKRRSKY |

Next, the peptides of Group 7 may be synthesized by substituting amino acid 3 of the peptide of SEQ ID NO: 1 with arginine, amino acid 4 with glutamine, amino acids 5 to 7 with lysine or arginine, amino acid 9 with tyrosine, and amino acid 10 with lysine (Table 7).

**[Table 7]**

| SEQ ID NO. | Amino acid sequence (N-C) |
|---|---|
| 49 | KYRQRKKNYK |
| 50 | KYRQRKRNYK |
| 51 | KYRQRRKNYK |
| 52 | KYRQRRRNYK |
| 53 | KYRQKKKNYK |
| 54 | KYRQKRKNYK |
| 55 | KYRQKKRNYK |
| 56 | KYRQKRRNYK |

Next, the peptides of Group 8 may be synthesized by substituting amino acid 3 of the peptide of SEQ ID NO: 1 with arginine, amino acid 4 with glutamine, amino acids 5 to 7 with lysine or arginine, amino acid 8 with serine, amino acid 9 with tyrosine, and amino acid 10 with lysine (Table 8).

**[Table 8]**

| SEQ ID NO. | Amino acid sequence (N-C) |
|---|---|
| 57 | KYRQRKKSYK |
| 58 | KYRQRKRSYK |
| 59 | KYRQRRKSYK |
| 60 | KYRQRRRSYK |
| 61 | KYRQKKKSYK |
| 62 | KYRQKRKSYK |
| 63 | KYRQKKRSYK |
| 64 | KYRQKRRSYK |

Next, the peptides of Group 9 may be synthesized by substituting amino acid 3 of the peptide of SEQ ID NO: 1 with lysine, amino acid 4 with glutamine, and amino acids 5 to 7 with lysine or arginine (Table 9).

**[Table 9]**

| SEQ ID NO. | Amino acid sequence (N-C) |
|---|---|
| 65 | KYKQRKKNKY |
| 66 | KYKQRKRNKY |
| 67 | KYKQRRKNKY |
| 68 | KYKQRRRNKY |
| 69 | KYKQKKKNKY |
| 70 | KYKQKRKNKY |
| 71 | KYKQKKRNKY |
| 72 | KYKQKRRNKY |

Next, the peptides of Group 10 may be synthesized by substituting amino acid 3 of the peptide of SEQ ID NO: 1 with lysine, amino acid 4 with glutamine, amino acids 5 to 7 with lysine or arginine, and amino acid 8 with serine (Table 10).

**[Table 10]**

| SEQ ID NO. | Amino acid sequence (N-C) |
|---|---|
| 73 | KYKQRKKSKY |
| 74 | KYKQRKRSKY |
| 75 | KYKQRRKSKY |
| 76 | KYKQRRRSKY |
| 77 | KYKQKKKSKY |
| 78 | KYKQKRKSKY |
| 79 | KYKQKKRSKY |
| 80 | KYKQKRRSKY |

Next, the peptides of Group 11 may be synthesized by substituting amino acid 3 of the peptide of SEQ ID NO: 1 with lysine, amino acid 4 with glutamine, amino acids 5 to 7 with lysine or arginine, amino acid 9 with tyrosine, and amino acid 10 with lysine (Table 11).

**[Table 11]**

| SEQ ID NO. | Amino acid sequence (N-C) |
|---|---|
| 81 | KYKQRKKNYK |
| 82 | KYKQRKRNYK |
| 83 | KYKQRRKNYK |

| | |
|---|---|
| 84 | KYKQRRRNYK |
| 85 | KYKQKKKNYK |
| 86 | KYKQKRKNYK |
| 87 | KYKQKKRNYK |
| 88 | KYKQKRRNYK |

Finally, the peptides of Group 12 may be synthesized by substituting amino acid 3 of the peptide of SEQ ID NO: 1 with lysine, amino acid 4 with glutamine, amino acids 5 to 7 with lysine or arginine, amino acid 8 with serine, amino acid 9 with tyrosine, and amino acid 10 with lysine (Table 12).

**[Table 12]**

| SEQ ID NO. | Amino acid sequence (N-C) |
|---|---|
| 89 | KYKQRKKSYK |
| 90 | KYKQRKRSYK |
| 91 | KYKQRRKSYK |
| 92 | KYKQRRRSYK |
| 93 | KYKQKKKSYK |
| 94 | KYKQKRKSYK |
| 95 | KYKQKKRSYK |
| 96 | KYKQKRRSYK |

As another aspect of the present invention, the present invention provides a polynucleotide encoding the peptide.

The polynucleotide may be mutated by substitution, deletion, insertion, or a combination thereof of one or more bases. When the nucleotide sequence is prepared by chemical synthesis, a synthetic method widely known in the art, for example, a method described in the document (Engels and Uhlmann, Angew Chem IntEd Engl., 37:73-127, 1988), may be used, and it may be synthesized using triester, phosphite, phosphoramidite, and H-phosphate methods, polymerase chain reaction (PCR) and other autoprimer methods, an oligonucleotide synthesis method on a solid support, and the like. For example, the polynucleotide encoding the peptide of the present invention may include the base sequence of SEQ ID NO: 4.

As another aspect, the present invention provides an expression vector including the polynucleotide, a transformant including the expression vector, and a method of preparing the peptide using the transformant.

The term "expression vector" of the present invention refers to a recombinant vector capable of expressing a target peptide in a target host cell, and means a genetic construct including essential regulatory elements operably linked to allow a gene insert to be expressed. The expression vector includes expression regulatory elements such as a start codon, a stop codon, a promoter, and an operator. The start codon and the stop codon are generally considered to be part of a nucleotide sequence encoding a polypeptide, and must be functional in an individual when the genetic construct is administered, and must be in frame with a coding sequence. The promoter of a vector may be constitutive or inducible.

The term "operably linked" of the present invention refers to a state in which a nucleic acid expression regulatory sequence and a nucleic acid sequence encoding a target protein or RNA are functionally linked to perform a general function. For example, a promoter and a nucleic acid sequence encoding a protein or RNA may be operably linked to affect the expression of the coding sequence. An operable link with an expression vector may be prepared using genetic recombination techniques well known in the art, and site-specific DNA cleavage and linkage may be done using enzymes generally known in the art.

**In** addition, the expression vector may include a signal sequence for release of the peptide to facilitate the isolation of the peptide from the cell culture medium. Specific initiation signals may also be required for efficient translation of the inserted nucleic acid sequence. These signals include the ATG start codon and adjacent sequences. In some cases, an exogenous translational regulation signal, which may include the ATG start codon, must be provided. These exogenous translational regulation signals and start codons may be from a variety of natural and synthetic sources. Expression efficiency may be increased by the introduction of appropriate transcriptional or translational enhancing factors.

**In** addition, the expression vector may further include a protein tag that may be optionally removed using an endopeptidase in order to facilitate detection of the peptide.

The term "tag" of the present invention refers to a molecule exhibiting a quantifiable activity or property, and may be a fluorescent molecule including a chemical fluorophore (fluoracer) such as fluorescein, a polypeptide fluorophore such as a fluorescent protein (green fluorescent protein: GFP) or a related protein; or an epitope tag such as a Myc tag, a Flag tag, a histidine tag, a leucine tag, an IgG tag, or a streptavidin tag. In particular, when an epitope tag is used, a peptide tag preferably consisting of 6 or more amino acid residues, and more preferably consisting of 8 to 50 amino acid residues, may be used.

In the present invention, the expression vector may include a nucleotide sequence encoding a peptide that provides the effect of promoting the physiological mineralization of dentin of the present invention as described above. At this time, the vector used may preferably be plasmid DNA, phage DNA, or the like, but is not particularly limited thereto, and more preferably a commercially developed plasmid (pUC18, pBAD, pIDTSAMRT-AMP, etc.), an *E*. *coli*-derived plasmid (pYG601BR322, pBR325, pUC118, pUC119, etc.), a *Bacillus subtilis-*derived plasmid (pUB110, pTP5, etc.), a yeast-derived plasmid (YEp13, YEp24, YCp50, etc.), phage DNA (Charon4A, Charon21A, EMBL3, EMBL4, λgt10, λgt11, λZAP, etc.), an animal virus vector (retrovirus, adenovirus, vaccinia virus, etc.), or an insect virus vector (baculovirus, etc.) as long as the peptide may be produced. Since the expression vectors show different protein expression levels and modifications depending on the host cell, it is preferable to select and use a host cell that is most suitable for the purpose.

The transformant provided in the present invention may be produced by introducing the expression vector provided in the present invention into a host and transforming it, and may be used to produce the peptide by expressing the polynucleotide included in the expression vector. The transformation may be carried out by various methods, and a CaCl₂ precipitation method, the Hanahan method in which efficiency is increased by using a reducing substance called dimethyl sulfoxide (DMSO) in the CaCl₂ precipitation method, electroporation, a calcium phosphate precipitation method, a protoplast fusion method, a stirring method using silicon carbide fibers, an *Agrobacterium-*mediated transformation method, a transformation method using polyethylene glycol (PEG), and a dextran sulfate, lipofectamine, or drying/inhibition-mediated transformation method may be used, but the method is not particularly limited thereto as long as the peptide may be produced. In addition, the host used for producing the transformant may be a bacterial cell such as *Escherichia coli, Streptomyces,* or *Salmonella typhimurium;* a yeast cell such as *Saccharomyces cerevisiae* or *Schizosaccharomyces pombe;* a fungal cell such as *Pichia pastoris;* an insect cell such as *Drosophila* or *Spodoptera* Sf9 cell; an animal cell such as a Chinese hamster ovary (CHO) cell, a CV-1 in Origin with SV40 genes (COS) cell, a non-secreting murine myeloma (NS0) cell, a 293 cell, or a Bowes melanoma cell; or a plant cell, but is not also particularly limited thereto as long as the peptide may be produced.

The transformant may also be used in a method of producing a peptide that provides an effect of promoting the physiological mineralization of dentin of the present invention. Specifically, the method of producing a peptide that provides an effect of promoting the physiological mineralization of dentin of the present invention may include: (a) a step of culturing the transformant to obtain a culture; and (b) a step of recovering the peptide of the present invention from the culture.

The term "culturing" of the present invention refers to a method of growing microorganisms under appropriately artificially controlled environmental conditions. In the present invention, the method of culturing a transformant may be performed using a method widely known in the art. Specifically, the culturing may be performed in a continuous manner in a batch process, or a fed batch or repeated fed batch process, but is not particularly limited thereto as long as a peptide that provides an effect of promoting the physiological mineralization of dentin of the present invention may be expressed and produced.

The medium used for cultivation should satisfy the requirements of a specific strain in an appropriate manner while the temperature, pH, and the like are adjusted under aerobic conditions in a common medium containing a suitable carbon source, nitrogen source, amino acids, vitamins, and the like. As a carbon source that may be used, a mixed sugar of glucose and xylose is used as a main carbon source, and in addition, sugars and carbohydrates such as sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid are included. These substances may be used individually or as a mixture. As a nitrogen source that may be used, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, and peptone, NZ-Amine, meat extract, yeast extract, malt extract, corn steep liquor, casein hydrolysate, fish or a decomposition product thereof, and defatted soybean cake or a decomposition product thereof may be used. These nitrogen sources may be used alone or in combination. The medium may contain monopotassium phosphate, dipotassium phosphate, and the corresponding sodium-containing salts as a phosphorus source. A phosphorus source that may be used includes potassium dihydrogen phosphate or dipotassium hydrogen phosphate or the corresponding sodium-containing salts. In addition, as inorganic compounds, sodium chloride, calcium chloride, iron chloride, magnesium sulfate, iron sulfate, manganese sulfate, and calcium carbonate may be used. Finally, in addition to the above-mentioned substances, essential growth substances such as amino acids and vitamins may be used.

In addition, suitable precursors may be used in the culture medium. The above-mentioned raw materials may be added to the culture in a batch, fed-batch, or continuous manner in an appropriate manner during the culture process, but are not particularly limited thereto. The pH of the culture may be adjusted by using a basic compound such as sodium hydroxide, potassium hydroxide, or ammonia, or an acid compound such as phosphoric acid or sulfuric acid in an appropriate manner.

**In** addition, a defoaming agent such as fatty acid polyglycol ester may be used to suppress bubble formation. Oxygen or an oxygen-containing gas (e.g., air) is injected into the culture to maintain an aerobic state. The temperature of the culture is usually 27 °C to 37 °C, preferably 30 °C to 35 °C. The culturing is continued until the maximum amount of the peptide is produced. This purpose is usually achieved in 10 to 100 hours.

**In** addition, the step of recovering the peptide from the culture may be performed by a method known in the art. Specifically, as the recovery method, a method such as centrifugation, filtration, extraction, spraying, drying, evaporation, precipitation, crystallization, electrophoresis, differential dissolution (e.g., ammonium sulfate precipitation), chromatography (for example, ion exchange, affinity, hydrophobicity, and size-exclusion) may be used, but the recovery method is not particularly limited thereto as long as it may be used to recover the produced peptide.

The term "prevention" of the present invention refers to all acts that inhibit or delay the occurrence of pulp necrosis, tooth extraction, or tooth loss, or secondary caries or pulpitis that may occur due to dental restorative materials, by administering the composition for dentin adhesion and the composition for pulp restoration including the peptide of the present invention.

The term "treatment" of the present invention refers to all acts of performing restorative treatment of a tooth by administering to an individual in need of restorative treatment a restorative material including the composition for dentin adhesion or the composition for dentin adhesion, or a composition for pulp restoration, each of which includes the peptide of the present invention as an active ingredient, in order to promote the physiological remineralization of dentin between the tooth and the restorative material.

The composition of the present invention may be prepared in the form of a composition for treating damaged dentin such as dental caries or fracture, which further includes a suitable carrier (natural or unnatural carrier), excipient or diluent commonly used in the preparation of the above-mentioned tooth restorative composition. Specifically, the composition may be formulated and used in the form of a sterile injectable solution that may be administered to a region where damaged dentin such as dental caries or fracture has occurred according to a conventional method. In the present invention, examples of the carriers, excipients, and diluents that may be included in the composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and collagen. When formulating, the composition may be prepared using diluents or excipients such as commonly used fillers, bulking agents, binders, wetting agents, disintegrating agents, and surfactants. In particular, sterilized aqueous solutions, non-aqueous solvents, suspensions, emulsions, lyophilized preparations, suppositories, ointments (e.g., pulp liners, etc.), and the like may be included. As a non-aqueous solvent and a suspension, propylene glycol, polyethylene glycol, vegetable oils such as olive oil, and injectable esters such as ethyl oleate may be used. As a base for suppositories, Witepsol, Macrogol, Tween 61, cacao oil, laurel oil, glycerol, gelatin or the like may be used.

The peptide included in the composition of the present invention may be included in an amount of 0.0001% to 50% by weight, more preferably 0.01% to 20% by weight, based on the total weight of the final composition, but the amount is not particularly limited thereto.

The composition of the present invention may be administered in a pharmaceutically effective amount. The term "pharmaceutically effective amount" of the present invention refers to an amount sufficient to treat or prevent a disease at a reasonable benefit/risk ratio applicable to medical treatment or prevention, and the effective dosage level can be determined according to the severity of the disease, the activity of the drug, the patient's age, weight, health, sex, the patient's sensitivity to the drug, the administration time, administration route, and excretion rate of the composition of the present invention used, treatment period, the drug used in combination or simultaneously with the composition of the present invention used, and other factors well known in the medical field. The composition of the present invention may be administered alone or in combination with a known composition for treating damaged dentin such as dental caries and fracture. It is important to administer an amount that enables to obtain the maximum effect with the minimum amount without side effects by considering all of the above-mentioned factors.

The dosage of the composition of the present invention may be determined by a person skilled in the art in consideration of the purpose of use, the severity of the disease, the patient's age, weight, sex, medical history, or the type of substance used as the active ingredient. For example, the composition of the present invention may be administered to an adult in an amount of about 0.1 ng to about 100 mg/kg, preferably 1 ng to about 10 mg/kg, and the composition of the present invention may be administered once a day or administered several times in divided doses, but the frequency of administration is not particularly limited thereto. The above dosage does not limit the scope of the present invention in any way.

As another aspect of the present invention, the present invention provides a method of treating damaged dentin such as dental caries or fracture, including a step of administering the composition in a pharmaceutically effective amount to an individual suffering from damaged dentin such as dental caries or fracture.

The term "subject" of the present invention may include, without limitation, mammals such as mice and livestock that require treatment of damaged dentin such as dental caries or fracture, but may exclude humans from the individuals suffering from the disease.

The composition for treating damaged dentin such as dental caries and fracture of the present invention may be administered via any general administration routes as long as the composition may reach the target tissue. The composition of the present invention may be provided in any formulation suitable for topical application depending on the purpose, but is not particularly limited thereto. For example, it may be administered orally, transdermally, intravenously, intramuscularly, or subcutaneously. The composition may be an injection, a solution for external use on the skin, a suspension, an emulsion, a gel, a patch, or a spray, but is not limited thereto. The formulation may be easily prepared according to a conventional method in the relevant field, and a surfactant, an excipient, a hydrating agent, an emulsifying agent, a suspending agent, a salt or buffer for osmotic pressure control, a coloring agent, a flavoring agent, a stabilizer, an antiseptic, a preservative, or other commonly used auxiliary agents may be appropriately used.

As still another aspect, the present invention provides a quasi-drug composition for preventing or ameliorating damaged dentin such as dental caries and fracture, containing the peptide.

The term "amelioration" of the present invention refers to all acts of at least reducing a parameter related to a condition to be treated, for example, the degree of symptoms.

In the present invention, the amelioration may be interpreted as all acts of ameliorating or beneficially improving symptoms of damaged dentin such as dental caries and fracture, by administering a composition containing the peptide of the present invention as an active ingredient to an individual in need of treatment of damaged dentin such as dental caries and fracture in order to promote the synthesis of dentin.

The term "quasi-drug" of the present invention refers to products that are used for the purpose of diagnosing, treating, ameliorating, alleviating, managing, or preventing human or animal diseases, and have a milder effect than medicines. For example, according to the Pharmaceutical Affairs Act, quasi-drugs are products other than those used for the medicinal purposes and include fibers and rubber products used for the treatment or prevention of human or animal diseases, products that have a mild or no direct effect on the human body and those similar thereto that are not an instrument or machine, and sterilizers and insecticides used to prevent infectious diseases.

In the present invention, the type or formulation of the quasi-drug composition containing the peptide is not particularly limited, but may be, for example, a skin or hair disinfectant, a skin or hair cleaning product, soap, shampoo, skin ointment, and the like.

As yet another aspect, the present invention provides a health functional food composition for preventing or ameliorating damaged dentin such as dental caries and fracture, containing the peptide.

The term "food" of the present invention includes meat, sausage, bread, chocolate, candies, snacks, confectioneries, pizza, ramen, other noodles, gum, dairy products including ice cream, various soups, beverages, tea, drinks, alcoholic beverages, vitamin supplements, health functional foods, and health food, and includes all foods in the conventional sense.

The "health functional food" is the same term as food for special health use (FoSHU), and refers to food with a high medicinal and healthcare effect that is processed to efficiently exhibit bioregulatory functions in addition to supplying nutrients. Here, "functionality" refers to regulating nutrients for the structure and function of the human body or obtaining a useful effect for health purposes such as physiological effects. The food of the present invention may be manufactured by a method commonly used in the art, and may be manufactured by adding raw materials and ingredients commonly added in the art during the manufacturing process. In addition, the food may be manufactured into any formulation without limitation as long as it is a formulation recognized as food. The food composition of the present invention may be manufactured in various forms of formulations, and unlike general drugs, it has the advantage of having no side effects that may occur when taking drugs for a long time because food is used as a raw material, and has excellent portability, and therefore the food of the present invention may be consumed as a supplement to enhance the effect of preventing or ameliorating damaged dentin such as dental caries and fracture.

The health food refers to food that has a more active health maintenance or promotion effect than general food, and health supplement food refers to food for the purpose of health supplementation. In some cases, the terms health functional food, health food, and health supplement food may be used interchangeably.

Specifically, the health functional food refers to a food product manufactured by adding the peptide of the present invention to food materials such as beverages, tea, spices, gum, and confectioneries, or by manufacturing it in the form of capsules, powder, suspension, and the like, and which has a specific health effect when consumed. However, since the health functional food is manufactured by using food as raw materials, unlike general drugs, it has the advantage of not having side effects that may occur with long-term use of drugs.

The food composition of the present invention may be used very usefully because it can be consumed on a daily basis, and thus can be expected to exhibit a high effect on preventing or ameliorating damaged dentin such as dental caries and fracture.

The food composition may further include a physiologically acceptable carrier, and the type of carrier is not particularly limited, and any carrier commonly used in the art may be used.

In addition, the food composition may include additional ingredients commonly used in food compositions to improve odor, taste, appearance, and the like. For example, it may include vitamins A, C, D, E, B1, B2, B6, and B12, niacin, biotin, folate, pantothenic acid, and the like. In addition, it may include minerals such as zinc (Zn), iron (Fe), calcium (Ca), chromium (Cr), magnesium (Mg), manganese (Mn), copper (Cu), and chromium (Cr). It may also include amino acids such as lysine, tryptophan, cysteine, and valine.

In addition, the food composition may include food additives such as preservatives (potassium sorbate, sodium benzoate, salicylic acid, sodium dehydroacetate, etc.), bactericides (bleaching powder and high-grade bleaching powder, sodium hypochlorite, etc.), antioxidants (butylated hydroxyanisole (BHA), butylated hydroxytoluene (BHT), etc.), colorants (tar color, etc.), color developers (sodium nitrite, sodium acetate, etc.), bleaching agents (sodium sulfite), seasonings (monosodium glutamate (MSG), etc.), sweeteners (dulcin, cyclamate, saccharin, sodium, etc.), flavorings (vanillin, lactones, etc.), leavening agents (alum, D-potassium hydrogen tartrate, etc.), reinforcing agents, emulsifiers, thickeners (gelling agents), film-forming agents, gum bases, foam suppressants, solvents, and improvers. The additives may be selected depending on the type of food and used in an appropriate amount.

The peptide of the present invention may be added as is or used together with other food or food ingredients, and may be used appropriately according to a conventional method. The mixing amount of the active ingredient may be appropriately determined according to its purpose of use (prevention, health, or therapeutic treatment). In general, when manufacturing food or beverages, the food composition of the present invention may be added to the food or beverage in an amount of 50 parts by weight or less, specifically in an amount of 20 parts by weight or less. However, in case of long-term intake for the purpose of health and hygiene, an amount below the above-mentioned range may be included, and since there is no problem in terms of safety, the active ingredient may also be used in an amount above the above-mentioned range.

As an example, the food composition of the present invention may be used as a health beverage composition, and in this case, like a conventional beverage, various flavoring agents, natural carbohydrates, or the like may be contained as additional ingredients. The above-mentioned natural carbohydrates may be monosaccharides such as glucose and fructose; disaccharides such as maltose and sucrose; polysaccharides such as dextrin and cyclodextrin; sugar alcohols such as xylitol, sorbitol, and erythritol. As the sweetener, natural sweeteners such as thaumatin and stevia extract or synthetic sweeteners such as saccharin and aspartame may be used. The proportion of the natural carbohydrates may be generally about 0.01 to 0.04 g, specifically about 0.02 to 0.03 g per 100 mL of the health beverage composition of the present invention.

In addition to the above-mentioned ingredients, the health beverage composition may contain various nutrients, vitamins, electrolytes, flavoring agents, colorants, pectic acid, salts of pectic acid, alginic acid, salts of alginic acid, organic acids, protective colloid thickeners, pH regulators, stabilizers, preservatives, glycerin, alcohol, or carbonating agents. In addition, fruit pulp for the production of natural fruit juice, fruit juice beverages, or vegetable beverages may be contained. These ingredients may be used independently or in combination. The proportion of these additives is not particularly important, but is generally selected in the range of 0.01 to 0.1 parts by weight per 100 parts by weight of the health beverage composition of the present invention.

The food composition of the present invention may contain the peptide at various percentages by weight as long as it may exhibit the effect of preventing or ameliorating damaged dentin such as dental caries or fracture, and specifically, may contain the peptide of the present invention in an amount of 0.00001% to 100% by weight or 0.01% to 80% by weight based on the total weight of the food composition, but is not limited thereto.

### [Advantageous Effects]

The dentin adhesive according to an embodiment of the present invention can prevent or suppress secondary caries or pulpitis that may occur due to the dental restorative material by preventing or suppressing pulp necrosis, tooth extraction, or tooth loss that may occur when applying various dental restorative materials to teeth.

When the area of damage to the tooth is close to the pulp or has invaded the pulp, in the case in which the area of the exposed pulp is small, the composition for pulp restoration according to an embodiment of the present invention can be directly applied to seal and restore the pulp, which enables the sealing of the pulp and prevents or suppresses microleakage that may occur at the boundary of the restored dentin, thereby providing high reliability and sustainability for the treatment of tooth damage.

The effects of the present invention are not limited to those mentioned above, and other effects that are not mentioned may be clearly understood by those skilled in the art to which the present invention pertains, from the description below.

### [Description of Drawings]

FIG. 1 shows the effect of a mixed solution of the dentin adhesive and KH001 on the expression of marker genes for odontogenic differentiation of human pulp cells. The marker genes included DSPP, DMP1, and BSP, and the expression of the genes was measured by reverse transcription-polymerase chain reaction (RT-PCR). All values in the results below are expressed as the average and standard deviation of the results of triplicate experiments, and *p<0.05 indicates the results compared to the control.

FIG. 2 shows the results of measuring dentinal tubule permeability when a mixed solution of the dentin adhesive and KH001 was applied to human teeth with exposed dentin to examine whether KH001 reached the pulp well. FIG. 2A shows a schematic diagram illustrating the sample preparation process for measuring dentinal tubule permeability, and FIGS. 2B to 2D show the results of taking micrographs with a confocal microscope.

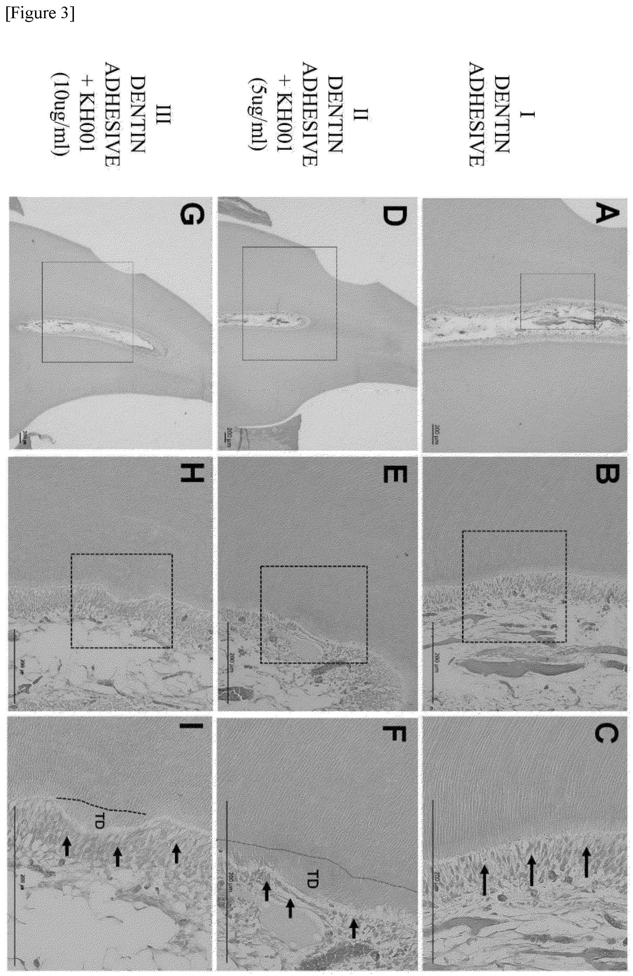

FIG. 3 shows the results of histologically evaluating the tubular dentin regeneration ability of a mixed solution of the dentin adhesive and KH001. After exposing the dentin by causing dish-shaped damage to the cervical region of a beagle's tooth, in the control, a dentin adhesive without KH001 was applied and then the dentin was restored with a resin, while in the experimental groups, a dentin adhesive containing KH001 was applied and then the dentin was restored with a resin. Thereafter, histological evaluation was carried out through hematoxylin and eosin (H&E) staining to investigate the formation of tubular dentin. The dotted line indicates the boundary of the newly formed tubular dentin, and TD (tertiary dentin) in the drawing refers to tubular dentin.

FIG. 4 shows the results of investigating the expression level of odontogenic differentiation marker genes in human pulp cells according to the concentration of KH001 under mineral trioxide aggregate (MTA)-conditioned medium conditions. The marker genes included DSPP, DMP1, and BSP, and the expression of the genes was measured by RT-PCR. All values in the results below are expressed as the mean and standard deviation of the results of triplicate experiments, and *p<0.05 and **p<0.01 indicate the results compared to the control.

FIG. 5 shows the results of investigating the changes in expression of odontogenic differentiation marker genes by differentiation date in order to confirm the efficacy of KH001 in a human pulp cell experiment in which an MTA-conditioned medium was treated.

FIG. 6 shows the results of investigating the changes in odontogenic differentiation and mineralization in order to confirm the efficacy of KH001 in a human pulp cell experiment in which an MTA-conditioned medium was treated.

FIG. 7 shows the results of investigating the changes in wound healing ability and cell migration ability in order to confirm the efficacy of KH001 in a human pulp cell experiment in which an MTA-conditioned medium was treated.

FIG. 8 shows micrographs taken with a confocal microscope to investigate the dentin tubule penetration ability of the composition for pulp restoration according to an embodiment of the present invention.

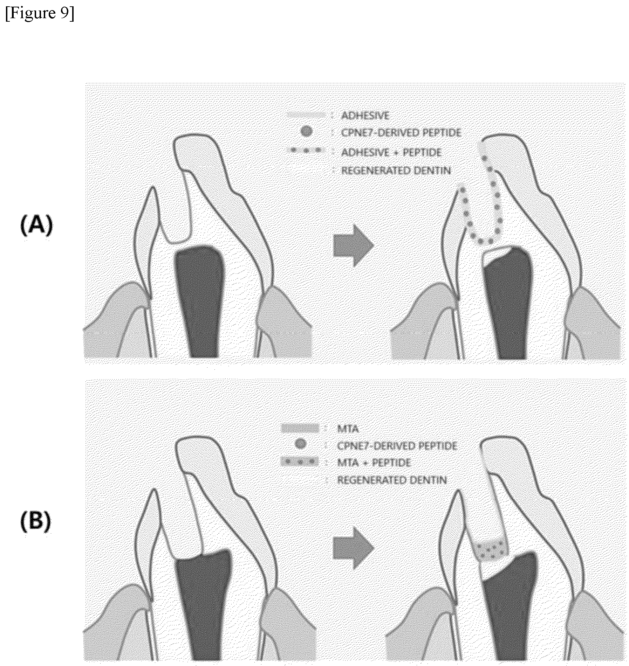

FIG. 9 shows a schematic diagram illustrating the areas where the composition for dentin adhesion and the composition for pulp restoration are applied in the process of treating damaged teeth and the appearance before and after the application.

### [Modes of the Invention]

The purpose and effects of the present invention and the technical configurations for achieving them will be clarified by referring to the examples described in detail below with the attached drawings. In explaining the present invention, when it is judged that a specific description of a known function or configuration may unnecessarily obscure the gist of the present invention, the detailed description thereof will be omitted. In addition, the terms described below are terms defined in consideration of the functions in the present invention, and these definitions may vary depending on the intention of the user or operator or customary practices.

However, the present invention is not limited to the examples disclosed below and may be implemented in various different forms. These examples are provided only to ensure that the disclosure of the present invention is complete and to fully inform a person having ordinary skill in the art to which the present invention pertains of the scope of the invention, and the present invention is defined only by the scope of the claims. Therefore, the definition should be made based on the contents throughout the present specification.

Hereinafter, examples of the present invention will be described in detail.

### Example 1: Materials and Methods

### Example 1-1: Preparation of peptides

The KH001 peptide (SEQ ID NO: 96) is a synthetic peptide corresponding to a 10 amino acid residue 344 to 353 fragment (KYKQKRRSYK) of the hCPNE7 protein. The KH001 peptide was synthesized using the 9-fluorenylmethoxycarbonyl (Fmoc)-based solid phase method. The purity of the synthesized peptide was 97% or higher as determined by high-performance liquid chromatography.

### Example 1-2: Cell culture

Mouse dental papilla cell-23 (MDPC-23: mouse pre-odontoblast cell line) cells were provided by Dr. J.E. Nor (University of Michigan, Ann Arbor, MI, USA) and cultured in Dulbecco's modified Eagle's medium (DMEM; Gibco BRL., Carlsbad, CA, USA), and C3H10T1/2 cells were obtained from the American Type Culture Collection (ATCC, Manassas, VA, USA) and cultured in Roswell Park Memorial Institute (RPMI) 1640 medium (Gibco BRL). Both cell lines were supplemented with 10% heat-inactivated fetal bovine serum (FBS; Gibco BRL) and antibiotic-antimycotic (Gibco BRL) at 37 °C in a 5% CO₂ atmosphere.

Cells were cultured in Minimum essential medium α (MEM-α; Gibco BRL) for use in *in vitro* and *ex vivo* experiments. For differentiation of human pulp cells (hDPCs) and MDPC-23 cells, 80% to 90% confluent cells were cultured in a medium supplemented with 5% FBS, ascorbic acid (50 µg/mL), and β-glycerophosphate (10 mM) for three weeks. Passages 2 to 4 were used for hDPSCs, and passages 23 to 25 were used for MDPC-23 cells.

### Example 2-1-1: Preparation of composition for dentin adhesion

KH001 and Bisco Dental's dentin adhesive, All-Bond Universal (ABU), were added at a ratio of 2 mg KH001 per 1 mL of ABU, and the resulting mixture was stirred under reduced pressure conditions (-750 mmHg) in a stirrer for about 40 minutes (stirring conditions: PADDLE 10 to 30 rpm, DISPERSE 500 to 600 rpm, HOMO 2400 to 3200 rpm). Thereafter, the resulting mixture was stored in a light-shielded container. The prepared composition for dentin adhesion was stored in a refrigerator at 4 °C for about three months after preparation and applied to Examples 2-3 and 2-4, which are shown in Table 13.

### Example 2-1-2: Preparation of composition for dentin adhesion

In the cell experiment on human pulp cells (hDPCs) prepared in Example 1-2, in order to prevent side effects such as cell death due to the toxicity of the dentin adhesive monomer, ABU was diluted with 10% α-MEM at a ratio of 1:5000 and used to prepare a composition for dentin adhesion. KH001 peptide powder was dissolved in the diluted solution so as to be contained at each of 5 µg/ml and 10 µg/ml, and the composition for dentin adhesion prepared in this manner was applied to Example 2-2. The prepared composition for dentin adhesion was stored in a refrigerator at 4 °C after preparation.

### Example 2-2: Analysis of regulation of odontogenic differentiation marker gene expression

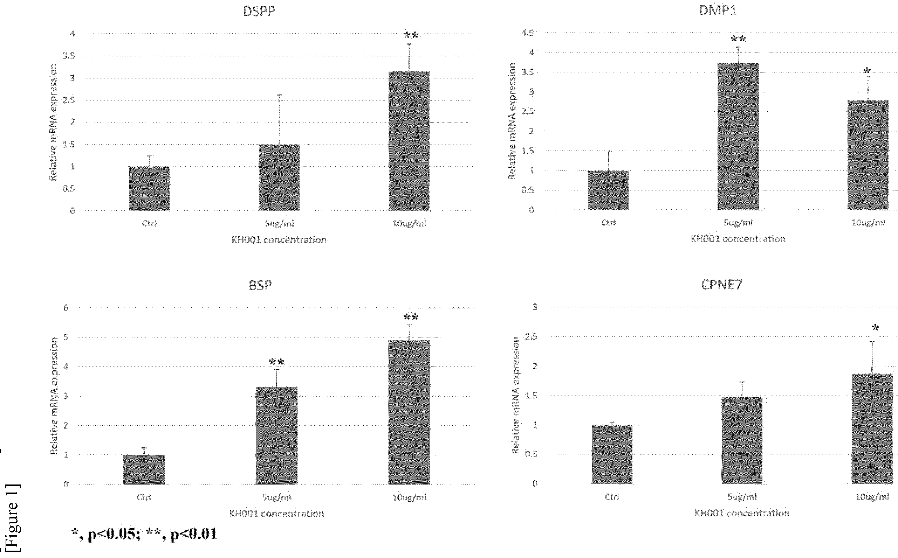

After treating the human pulp cells (hDPC) prepared in Example 1-2 with the composition for dentin adhesion of Example 2-1-2, the expression of DSPP, DMP1, and BSP genes was measured by reverse transcription-polymerase chain reaction (RT-PCR) and real-time PCR analysis. The gene expression values shown in FIG. 1 are expressed as the average and standard deviation of the results of triplicate experiments, and *p<0.05 and **p<0.01 indicate the results compared to the control.

Referring to FIG. 1, as the concentration of KH001 increased, the expression levels of odontogenic differentiation marker genes, DSPP, BSP, and DMP1, increased, and when KH001 was treated at a concentration of 10 µg/ml, the expression of DSPP, DMP1, and BSP mRNA increased by more than 2.5 times.

### Example 2-3: Observation of dentinal tubule permeability

The composition for dentin adhesion prepared according to Example 2-1-1 of the present invention was applied to extracted human teeth to observe dentinal tubule permeability. The extracted human teeth were impacted human third molars from patients aged 18 to 22, provided by the Seoul National University Dental Hospital. The experimental protocol was approved by the Institutional Review Board (IRB; IRB Number: S-D20140007), and prior consent was obtained from all patients.

The crown of the extracted human tooth was transversely cut using a diamond saw to expose the dentinal tubule, and then the exposed human tooth was treated. A composition for dentin adhesion prepared according to Example 2-1-1 containing the KH001 peptide (SEQ ID NO: 96), to which rhodamine was attached as a fluorescent dye, was applied to the coronal surface of the extracted human tooth.

The composition for dentin adhesion was applied without a brushing process or an air-drying process and left for about 30 seconds for photopolymerization, and then confocal images were taken.

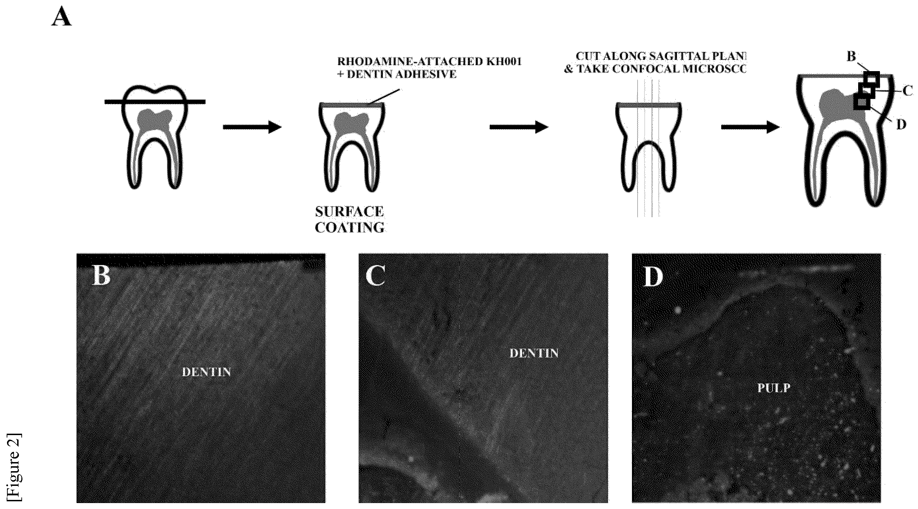

FIG. 2 shows the result of measuring the dentinal tubule permeability to examine whether KH001 reached the pulp well when a mixed solution of the dentin adhesive and KH001 was applied to the human tooth with exposed dentin for about 30 seconds. Referring to FIG. 2, FIG. 2A shows a schematic diagram illustrating the sample preparation process for measuring dentinal tubule permeability, and FIGS. 2B to 2D show the results of taking micrographs with a confocal microscope, and the observation results confirmed that the peptide KH001 flowed well into the pulp cavity along the dentinal tubule.

### Example 2-4. Histological evaluation of tubular dentin regeneration ability

After exposing dentin by causing dish-shaped damage to the cervical region of a beagle's tooth, in the control, a dentin adhesive without KH001 was applied and then the dentin was restored with a resin, while in the experimental groups, a dentin adhesive containing KH001 was applied and then the dentin was restored with a resin. Thereafter, histological evaluation was performed through hematoxylin and eosin (H&E) staining to investigate the formation of tubular dentin.

FIG. 3 shows the results of histologically evaluating the tubular dentin regeneration ability of the composition for dentin adhesion of Example 2-1-1. Referring to FIG. 3, the dotted line indicates the boundary of the newly formed tubular dentin, and TD (newly formed tertiary dentin) indicates the dentin with the newly formed dentinal tubules.

Referring to FIG. 3, in the control (I) in which only the dentin adhesive (ABU) was applied, tubular dentin was not formed below the dentin damage area, but in the experimental group (II) (containing **5 µg/ml of KH001)** and experimental group (III) (containing **10 µg/ml of KH001)** in which the composition for dentin adhesion containing KH001 prepared in Example 2-1-2 was applied, it was confirmed that tubular dentin was formed toward the pulp below the dentin damage area. In addition, odontoblasts were arranged in a fence-like shape in the pulp adjacent to the newly formed tubular dentin, indicating that it was an activated healthy pulp.

Table 13 shows the results of a shear bond strength test performed by using the Ultradent method to confirm the adhesive strength of the composition for dentin adhesion containing KH001 prepared in Example 2-1-1 to dentin. After smoothing the surface of dentin with 320-grit sandpaper and 600-grit sandpaper, the dentin adhesive containing ABU (Bisco) as the control and KH001 as the experimental group at a concentration of 2 mg/mL was applied by the self-etching method for about 10 to 15 seconds, and then photopolymerized with a light source of 1000 mW/cm² intensity and scrubbed, and then Duo-Link Universal^{™} (DLU, Bisco) was bonded by irradiating with a light source of the same intensity for 20 seconds. Thereafter, the specimens were stored in deionized water (DI water) at 37 °C, and then measurement was performed. Each group included ten samples.

**[Table 13]**

| Adhesive + Cement | SBS, Mpa (SD) |
|---|---|
| ABU + DLU | 34.20 (8.22) |
| ABU w/KH001 + DLU | 33.43 (8.36) |

Table 13 shows the adhesive strength of the composition for dentin adhesion prepared in Example 2-1-1. Referring to Table 13, even though KH001 was included at a high concentration of about 200 times the effective concentration, the adhesive strength of the composition for dentin adhesion was similar to that of conventional dentin adhesives. These results suggest that even when the composition for dentin adhesion includes KH001 at a concentration of about 2 mg/mL or less, it can provide an adhesive strength equivalent to that of a conventional dentin adhesive.

### Example 3-1: Preparation of composition for pulp restoration

MTA (ProRoot MTA; Dentsply Sirona, New York, PA, USA) was mixed on a sterilized glass plate using a metal slab in a powder/liquid ratio according to the manufacturer's instructions and cured at room temperature for one hour for complete curing.

After curing was completed, the MTA was ground in a sterilized mortar and pestle into a powder form, which was then soaked in a-MEM (MEM-α; Gibco BRL), which is a culture medium for human pulp cells, for 24 hours and stored at approximately 37 °C to produce a conditioned medium.

Thereafter, the conditioned medium diluted at a ratio of x1/5, and treated with KH001 at a concentration of 5 µg/mL and 10 µg/mL to prepare a composition for pulp restoration.

### Example 3-2: Evaluation of the expression level of odontogenic differentiation marker genes (DSPP, DMP1, BSP) and the CPNE7 gene according to the concentration of KH001 in the composition for pulp restoration

After the total RNA of human pulp cells was extracted using Tri-Reagent^{™}, cDNA was synthesized by treatment with reverse transcriptase and oligo-(dT) primers (Invitrogen).

For the gene amplification process, 1 µL of the reverse transcribed cDNA, primers corresponding to each gene, and SYBR Green Supermix were mixed in a 96-well plate, and a quick cycle was carried out according to the manufacturer's instructions.

The amount of PCR product was measured as a ratio to glyceraldehyde 3-phosphate dehydrogenase (GAPDH), and the relative expression levels were compared using the CT (comparative threshold cycle) method.

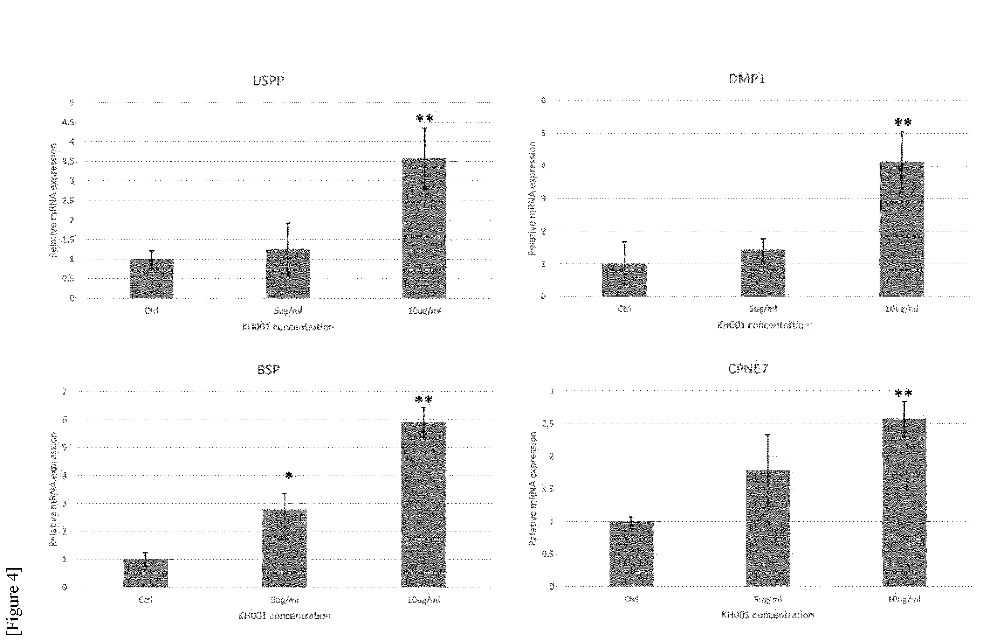

FIG. 4 shows the results of measuring the expression levels of the odontogenic differentiation marker genes and the CPNE7 gene according to the concentration of KH001 (KH001 under MTA-conditioned medium conditions) of the composition for pulp restoration in human pulp cells by RT-PCR. All the result values are expressed as the average and standard deviation of the results of triplicate experiments, and *p<0.05 and **p<0.01 indicate the results compared to the control.

### Example 3-3: Evaluation of the expression level of odontogenic differentiation marker genes (DSPP, DMP1, BSP) and the CPNE7 gene by date according to treatment of the composition for pulp restoration (KH001 10 µg/ml under MTA-conditioned medium conditions)

In order to compare and measure the function of the composition for pulp restoration containing KH001 10 **µg/ml** prepared by Example 3-1 in inducing the differentiation of human pulp cells into odontoblasts, a control (Ctrl) treated only with the differentiation medium, a group treated with the MTA-conditioned differentiation medium (MTA), and a group treated with the composition for pulp restoration (MTA-conditioned/CPNE7 functional peptide together, MTA+KH001) were compared.

The differentiation medium was basically prepared to contain 1% antibiotic, 10 mM beta-glycerophosphate, and 50 µg/mL ascorbic acid in α-MEM supplemented with 5% FBS.

After treating human pulp cells with each medium, RT-PCR was performed on days 0, 7, and 14 to examine the changes in expression of odontogenic differentiation marker genes.

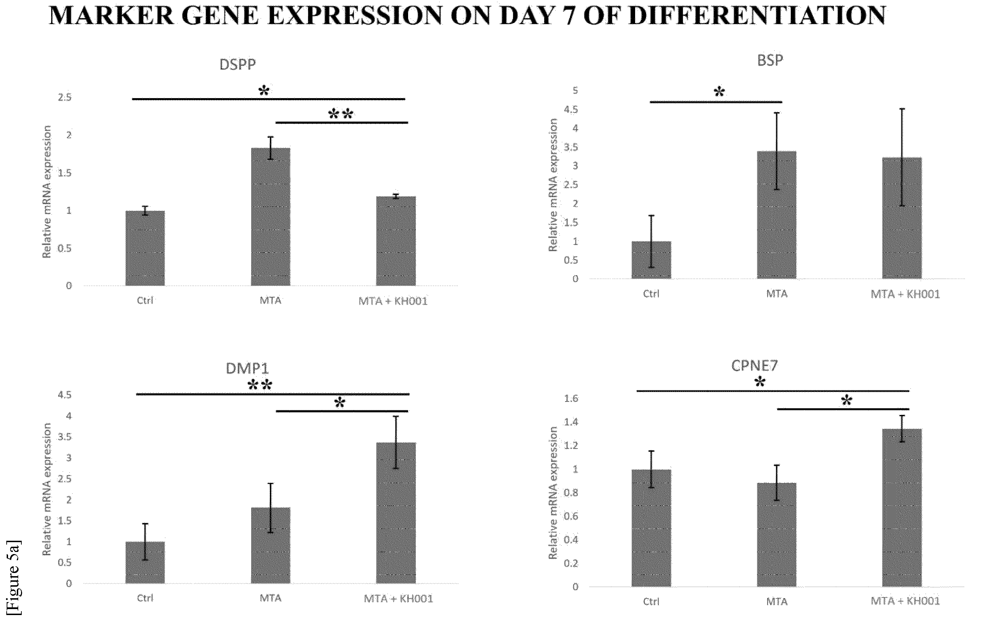

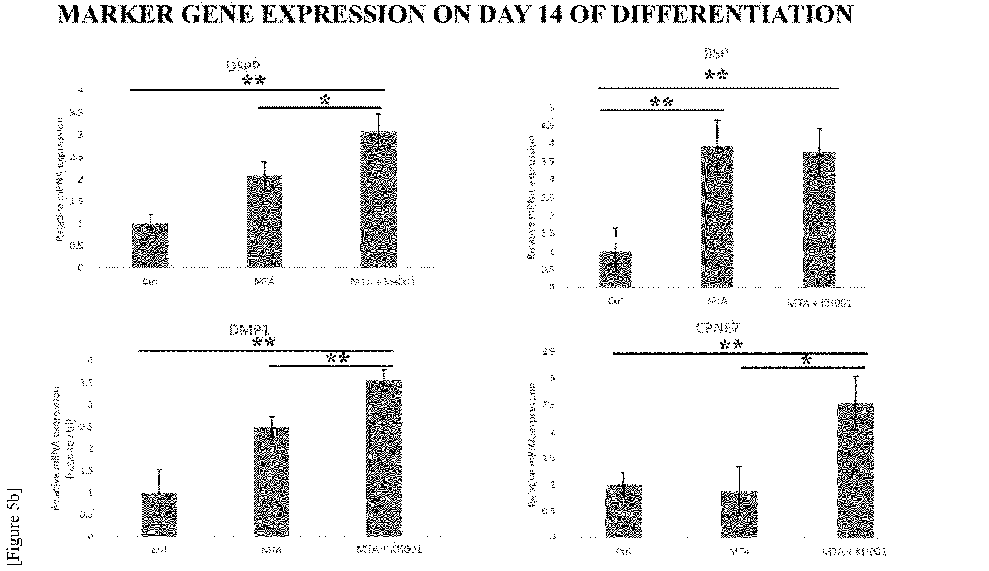

FIG. 5 shows the results of investigating the changes in expression of the odontogenic differentiation marker genes by differentiation date in order to confirm the efficacy of KH001 (MTA+KH001) in the experiments with human pulp cells treated with an MTA-conditioned medium. MTA is a preparation applied when the pulp is exposed or when the pulp is not exposed but only a thin layer of dentin remains and therefore damage to the pulp is expected. Therefore, odontoblasts originally present in the exposed pulp area are likely to die, and in order to recover these and form tertiary dentin in the exposed pulp area, it is important to induce the pulp cells to differentiate into odontoblasts.

Referring to FIG. 5, when investigating the expression of the odontoblast markers and mineralization-related gene during the differentiation process of the human pulp cells, the expression levels of DMP1 and CPNE7 in the group treated with MTA and the CPNE7 functional peptide together significantly increased compared to the group treated with MTA alone on day 7 of differentiation. However, on day 14, it was observed that the expression of DSPP, DMP1, and CPNE7 genes was significantly higher in the group treated with MTA and the CPNE7 functional peptide together than in the group treated with MTA only. BSP, which is a gene involved in the formation of bone-like dentin, did not increase significantly compared to the group treated with MTA only on both days 7 and 14, and was expressed at the equivalent level to the group treated with MTA only.

### Example 3-4: Evaluation of odontogenic differentiation and mineralization ability according to MTA-conditioned medium treatment

To investigate the effect of the CPNE7 functional peptide on the odontogenic differentiation and mineralization ability of odontoblasts in an environment treated with MTA, Alizarin red staining (ARS) was performed.

After human pulp cells (passage 2 to 4) were plated at a density of 1 × 10⁵ cells/well on a 6-well plate, when the cells reached approximately 80% confluency, the differentiation medium corresponding to each control and experimental group was treated for 7, 14, and 21 days, and the differentiation medium was exchanged with a fresh medium every 2 days.

To fix the cells, cells were treated with 4% paraformaldehyde at 4 °C for 15 minutes and treated with 40 mM Alizarin red S at room temperature for 30 minutes to stain the mineralization product using an Alizarin red S staining quantification kit.

Thereafter, for a quantitative analysis, cells were treated with 10% acetic acid at room temperature for 30 minutes, centrifuged, and 500 µL of the supernatant was extracted, and 200 µL of 10% ammonium hydroxide was added for neutralization.

Thereafter, the absorbance was measured at 405 nm with the ARS standard, and all experiments were performed in triplicate.

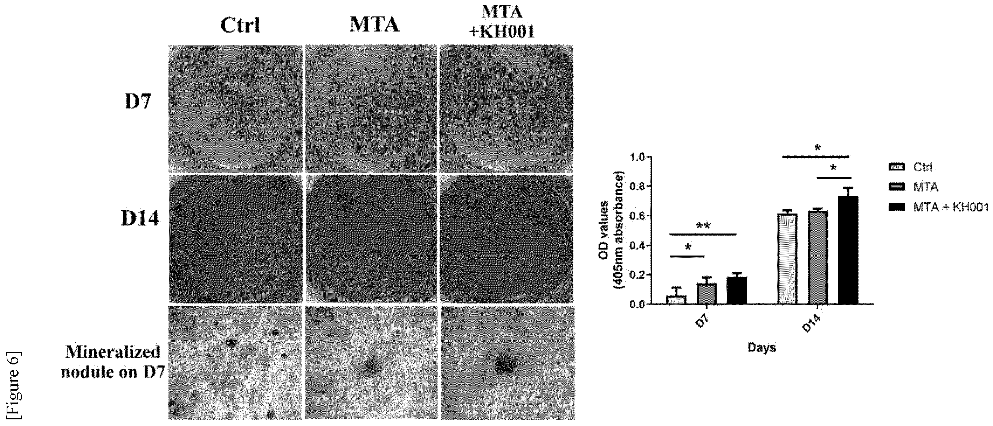

FIG. 6 shows the results of investigating the changes in odontogenic differentiation and mineralization in the experiment in which the human pulp cells were treated with the MTA-conditioned media to confirm the efficacy of KH001 (composition for pulp restoration).

The clinical situation in which MTA is applied is when the pulp is exposed and odontoblasts have died. In order to maintain the pulp in this situation, it is necessary to induce the differentiation of pulp cells into odontoblasts and for the differentiated odontoblasts to secrete mineralizing substances well. Through this process, the exposed pulp area may be filled with tertiary dentin, and MTA is considered a bioactive substance because it induces the formation of tertiary dentin. However, it can be seen that MTA induces the formation of bone-like restorative dentin, unlike physiological dentin or reactive dentin with dentinal tubules.

Referring to FIG. 6, it can be confirmed that the absorbance increased statistically significantly in the group treated with both MTA and KH001 than in the control at both time points of day 7 and day 14. It can be seen that on day 14, the mineralization level of the group treated with both MTA and KH001 showed a statistically significant difference compared to the group treated with only MTA.

### Example 3-5. Evaluation of wound healing ability and cell migration ability according to treatment with the composition for pulp restoration

Human pulp cells (passage number 2 to 4) were plated at a density of 1 × 10⁵ cells/well on a 6-well plate and cultured in 10% α-MEM.

The day after plating the cells, the central area of each well was scraped using a 200 µL pipette tip to create a cell-free 'wound area.'

After 24 hours, micrographs were taken using a 10x magnification objective lens of an upright microscope, and each micrograph was analyzed using the ImageJ program. All experiments were performed in triplicate.

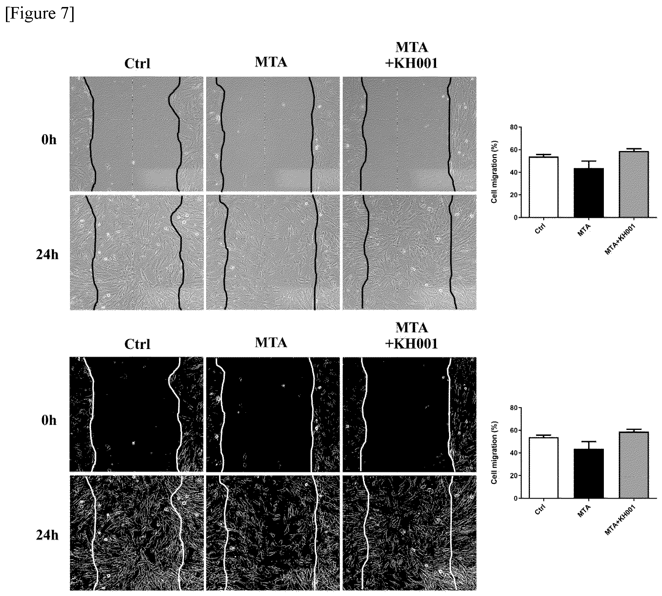

FIG. 7 shows the results of investigating the changes in the wound healing ability and cell migration ability in the experiment in which human pulp cells were treated with the MTA-conditioned medium to confirm the efficacy of KH001.

The pulp restoration technique using MTA is applied when odontoblasts and pulp cells are damaged. This means a condition in which there is a kind of 'wound' in the pulp, and the process of adjacent cells gathering to the area where cells are lost or damaged must precede the pulp restoration. Therefore, as a cell experiment model mimicking this process, a wound healing model was employed to investigate the changes in the wound healing ability and cell migration ability due to the composition for pulp restoration.

Referring to FIG. 7, a wound healing analysis was performed to determine the effect of KH001 on the wound healing and cell migration ability of odontoblasts in an environment treated with MTA. As a result, although it was not statistically significant, the group treated with MTA tended to have lower cell migration ability than the control (Ctrl). On the other hand, the group treated with both MTA and KH001 showed that the cell migration ability tended to recover to a level similar to the control. These results indicate that it is more advantageous to apply a product containing both MTA and KH001 rather than applying only MTA in a situation in which the pulp is exposed.

### Example 3-6. Evaluation of the dentin tubular penetration ability of the composition for pulp restoration (MTA prototype)

### A. Preparation of beagles

A total of 10 beagles (two years old) were used for the *in vivo* test. Depending on the periodontal conditions of each beagle, four to six maxillary premolars and six mandibular premolars were used.

The control and the experimental group were assigned to each beagle to eliminate errors that may occur due to individual nutrition, behavior, and pulp conditions. All experiments using animals were performed according to the protocol approved by the Animal Management Committee at the Seoul National University Veterinary Medical Teaching Hospital (SNU-180416-2-1, SNU-171020-5-2).

### B. Evaluation of cell penetration ability

A liquid containing 10 µg/mL CPNE7 functional peptide (KH001) was mixed *in vivo* with the ENDOCEM MTA powder according to the manufacturer's instructions, and then the resulting mixture was applied to the cervical region close to the pulp of a beagle's tooth to investigate the dentin tubule penetration ability of the composition for pulp restoration (MTA prototype).

A dish-shaped Class V cavity was formed in the cervical region of a beagle with a depth equal to half the diameter of a #4 round bur, and MTA containing the CPNE7 functional peptide (KH001) linked to fluorescent rhodamine was applied and fixed with 4% PFA, and specimens with a thickness of 0.1 mm in the sagittal direction were prepared using a digital low-speed diamond cutter.

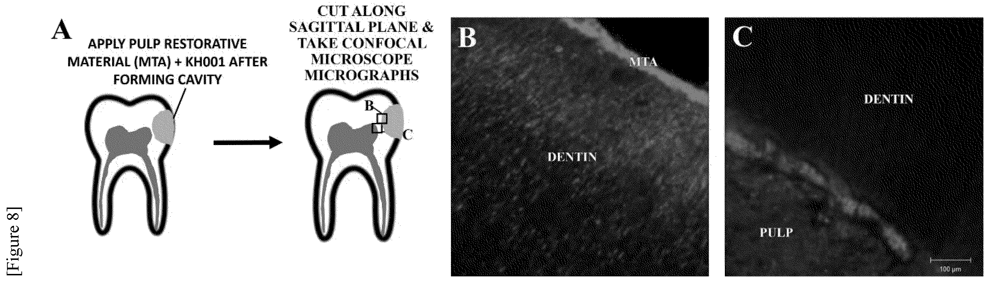

FIG. 8 shows micrographs taken with a confocal microscope to investigate the dentin tubule penetration ability of the composition for pulp restoration. FIG. 8A shows a schematic diagram of the sample preparation process, and FIGS. 8B and 8C show the results of examining whether KH001 reached the dentin and pulp, respectively.

Referring to FIG. 8, it can be seen that when 10 µg/mL of the CPNE7 functional peptide was mixed with the ENDOCEM MTA powder, the CPNE7 functional peptide flowed well along the dentinal tubule and reached the pulp.

Even though the dentin was much thicker than the actual case of applying MTA, in which the pulp is exposed or the remaining dentin is so thin that it is close to the pulp, the CPNE7 functional peptide reached the pulp well through the dentinal tubule. Therefore, it can be expected that the composition for pulp restoration with the applied CPNE7 functional peptide will seal the exposed pulp and provide effective restoration when performing pulp restoration.

This study was supported by the National Research and Development Project (1711138080 and 1711138079; "Development of Next-Generation Dental Restorative and Adhesive Materials Inducing Tooth Dentin Regeneration") of the Ministry of Science and ICT, the Ministry of Health and Welfare, and the Ministry of Trade, Industry and Energy (Korea Planning & Evaluation Institute of Industrial Technology: KEIT) in 2021.

The present specification and drawings have disclosed preferred embodiments of the present invention, and although specific terms have been used, they have been used only in a general sense to easily explain the technical contents of the present invention and to help understand the invention and are not intended to limit the scope of the present invention. It will be obvious to those skilled in the art to which the present invention pertains that other modifications based on the technical idea of the present invention may be implemented in addition to the embodiments disclosed herein.

## Claims

1. A composition for dentin adhesion, comprising a peptide consisting of an amino acid sequence of General Formula 1 below:
K-Y-R1-R2-R3-R4-R5-R6-R7-R8 (General Formula 1),
in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

2. The composition of claim 1, wherein the composition for dentin adhesion has any one amino acid sequence of SEQ ID NOs: 1 to 96.

3. A composition for pulp restoration, comprising mineral trioxide aggregate (MTA) and a peptide consisting of an amino acid sequence of General Formula 1 below:
K-Y-R1-R2-R3-R4-R5-R6-R7-R8 (General Formula 1),
in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

4. The composition of claim 3, wherein the composition for pulp restoration has any one amino acid sequence of SEQ ID NOs: 1 to 96.

5. A polynucleotide encoding the peptide of claim 1 or claim 3.

6. An expression vector comprising the polynucleotide of claim 5.

7. The composition of claim 1, wherein the composition includes a polypeptide in which the peptide is repeatedly linked.

8. The composition of claim 1, wherein the composition for dentin adhesion is used to bond a dental restorative material to damaged dentin.

9. The composition of claim 8, wherein the damaged dentin is dental caries or fractured dentin.

10. The composition of claim 1, further comprising a pharmaceutically acceptable carrier, excipient or diluent.

11. A method of improving or treating damaged dentin by administering the composition of claim 1 or claim 3 to an individual other than a human to prevent necrosis of dentin, tooth extraction, or tooth loss after treatment.
